# EUROPEAN PATENT SPECIFICATION

(11) **EP 1 286 965 B1**
(45) Date of publication and mention of the grant of the patent: **14.01.2004**
(21) Application number: 00927979.5
(22) Date of filing: 12.05.2000
(51) Int. Cl.: C07D 211/22, C07D 405/12

(54) **PIPERIDINE COMPOUNDS AND PROCESS FOR PROVIDING SUCH**
PIPERIDINVERBINDUNGEN UND VERFAHREN ZU DEREN HERSTELLUNG
COMPOSES DE PIPERIDINE ET PROCESSUS DE PRODUCTION CORRESPONDANT

(43) Date of publication of application: 05.03.2003
(73) Proprietor: Synthon B.V., 6545 CM Nijmegen (NL)
(72) Inventor: PETERS, Theodorus, Hendricus, Antonius, NL-6814 JB Arnhem (NL); LEMMENS, Jacobus, Maria, NL-6585 KM Mook (NL); SLANINA, Pavel, 664 31 Lelekovice 458 (CZ)
(74) Representative: Prins, Hendrik Willem
(86) International application number: PCT/NL2000/000321
(87) International publication number: WO 2001/085689

(56) References cited:
- EP-A- 0 190 496
- EP-A- 0 812 827
- WO-A-98/01424
- US-A- 5 874 447
- PATENT ABSTRACTS OF JAPAN vol. 2000, no. 07, 29 September 2000 (2000-09-29) & JP 2000 095780 A (ASAHI GLASS CO. LTD;), 4 April 2000 (2000-04-04)
- K. WILLCOCKS ET AL.: "The synthesis of [14C]-3S,4R-4-(4-fluorophenyl)-3-(3,4-meth ylenedioxyphenoxymethyl)piperidine hydrochloride (BRL 29060A), and mechanistic studies using carbon-13 labelling" JOURNAL OF LABELLED COMPOUNDS AND RADIOPHARMACEUTICALS, vol. XXXIII, no. 8, 1993, pages 783-794, XP000645839

## Description

The present invention relates to a piperidine compound useable in producing paroxetine, and to a process for providing this compound and paroxetine.

(-)Trans-4-(p-fluorophenyl) -N-methyl- piperidine 3-carbinol of the general formula (1) 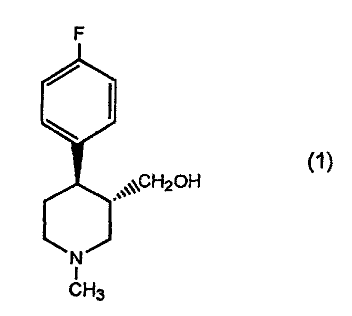 is a starting material in the synthesis of the pharmaceutically active compound paroxetine represented by the formula (2).

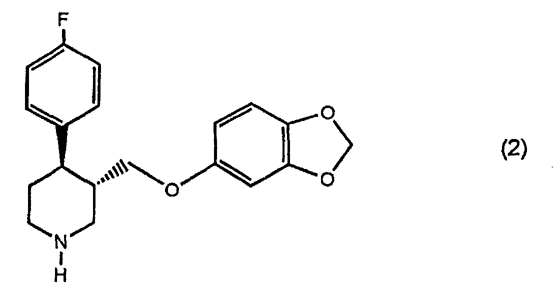

A known and useful synthetic procedure leading to paroxetine, formula (2), comprises a sequence of the following steps :
- The -OH group of compound (1) is substituted by a leaving group L;
- The reactive substrate (3) so produced reacts with an anion of sesamol (3,4-methylenedioxyphenol) to yield N-methylparoxetine (4)
- To remove the methyl group, the compound (4) is reacted with a chloroformate Cl-COOR to yield a carbamate compound (5). Several chloroformates with different R groups have been disclosed in the prior art as suitable;

The carbamate group in (5) is hydrolysed to paroxetine (2) in a strongly alkaline or acidic medium, dependent on the nature of the R substituent. For R= phenyl, alkaline conditions are preferred.

The general procedure is outlined in the following scheme 1.

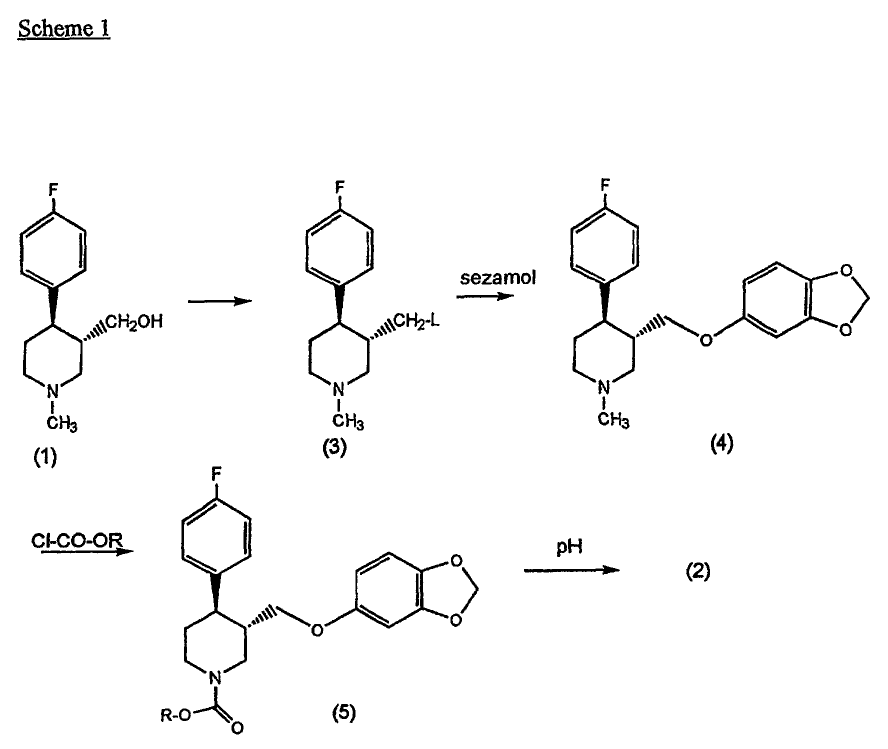

The leaving group L is so selected that it is reactive in the subsequent synthetic step. As leaving groups a chlorine atom or a sulfonic acid ester group such as mesyloxy or besyloxy group is known. A preferred compound (3) is the compound wherein the L group represents a mesyloxy group or a besyloxy group as these can be produced in good yields and without the use of corrosive and irritating substances such as thionylchloride.

The known intermediates (3), according to the the prior art are prepared in situ to be directly used in the reaction with sesamol. In attempts to isolate the product, i.e.after elaboration of reaction mixtures and evaporation of the solvent, the mesyloxy or besyloxy compound results in an oil. This has two serious disadvantages, namely:
1) Oily materials, produced in a synthesis, are likely to be contaminated with solvent and reagent residue from the synthesis which may cause side reactions in subsequent steps.
2) As a result of the production procedure, the starting carbinol (1) is usually contaminated with the corresponding des-fluoro impurity. If so, the process leading to the known intermediates (3) and the known way of elaboration of any of these intermediates (3) does not provide the intermediate (3) in desired purity. As a result, the produced paroxetine also contains an undesirably high amount of the des-fluoroparoxetine. If paroxetine is used as a pharmaceutical substance, the content of the des-fluoro paroxetine therein should be less than 0.1 % according to Pharmacopoeia prescriptions (e.g. Paroxetine hydrochloride USP XXIII). Purifying of crude paroxetine contaminated with the des-fluoroparoxetine is ineffective and uneconomical.

According to a first aspect of the present invention there is provided a process according to the claims 1-12 for providing and processing further a compound of formula (6), see below.

Compound (6) is isolateable in a solid form and accordingly has good handling properties, and is stable during prolonged storage and/or transport.

Furthermore this solid form is easily obtainable with a low content of the corresponding des-fluoro impurity, and/or is easily purified. The solid state provides advantages for transport sampling and weighing during industrial chemical production as well as for identification, analytical and purification procedures, which are extremely important in the production of pharmaceuticals.

The compound of formula (6) is preferably provided by contacting the compound of formula (1) with p-toluenesulfonylchloride under presence of an organic base, in an inert solvent in which the product is insoluble after cooling, followed by precipitation.

The compound of formula (6) can preferably be purified by crystallisation from an organic, water miscible solvent, preferably from an alkanol, whereby the content of the des-fluoroimpurity decreases under the level of about 0.2%.

The compound (6) can subsequenly be converted to paroxetine if desired.

In another aspect of the present invention, there is provided (-)trans 4-(p-fluorophenyl)-3-(p-toluenesulfonyloxymethyl)-N-methylpiperidine of the formula (6).

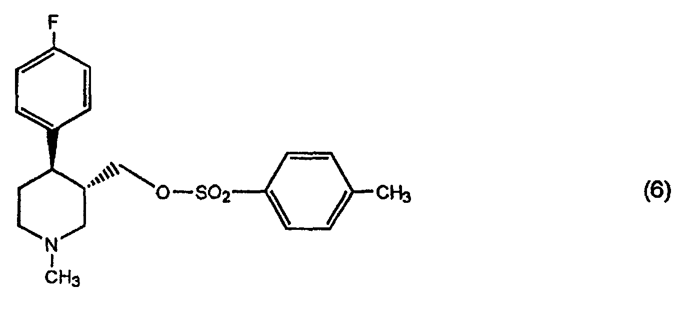

It is apparent that the formula (6) represents a specific case of a compound of general formula (3), wherein L represents a p-toluenesulfonyloxy (tosyloxy) group.

The compound of the formula (6) is particularly useful whenever it is isolated in a solid state, especially in a crystalline state.

In a preferred embodiment, the compound of the formula (6) contains less than about 0.2% of the structurally related des-fluoroimpurity.

Further characteristics of this compound and related compounds are to be found in the claims 13-20.

Further aspects of the present invention are to be found in claims 19-22.

Still another aspect of the present invention is a process for converting the compound (6) to paroxetine as well as the use of compound of formula (6) within the said process.

The invention will now be further clarified by way of the following description and examples.

The following scheme 2 illustrates a detailed process leading to paroxetine (isolated as paroxetine mesylate) comprising steps according to the present invention:

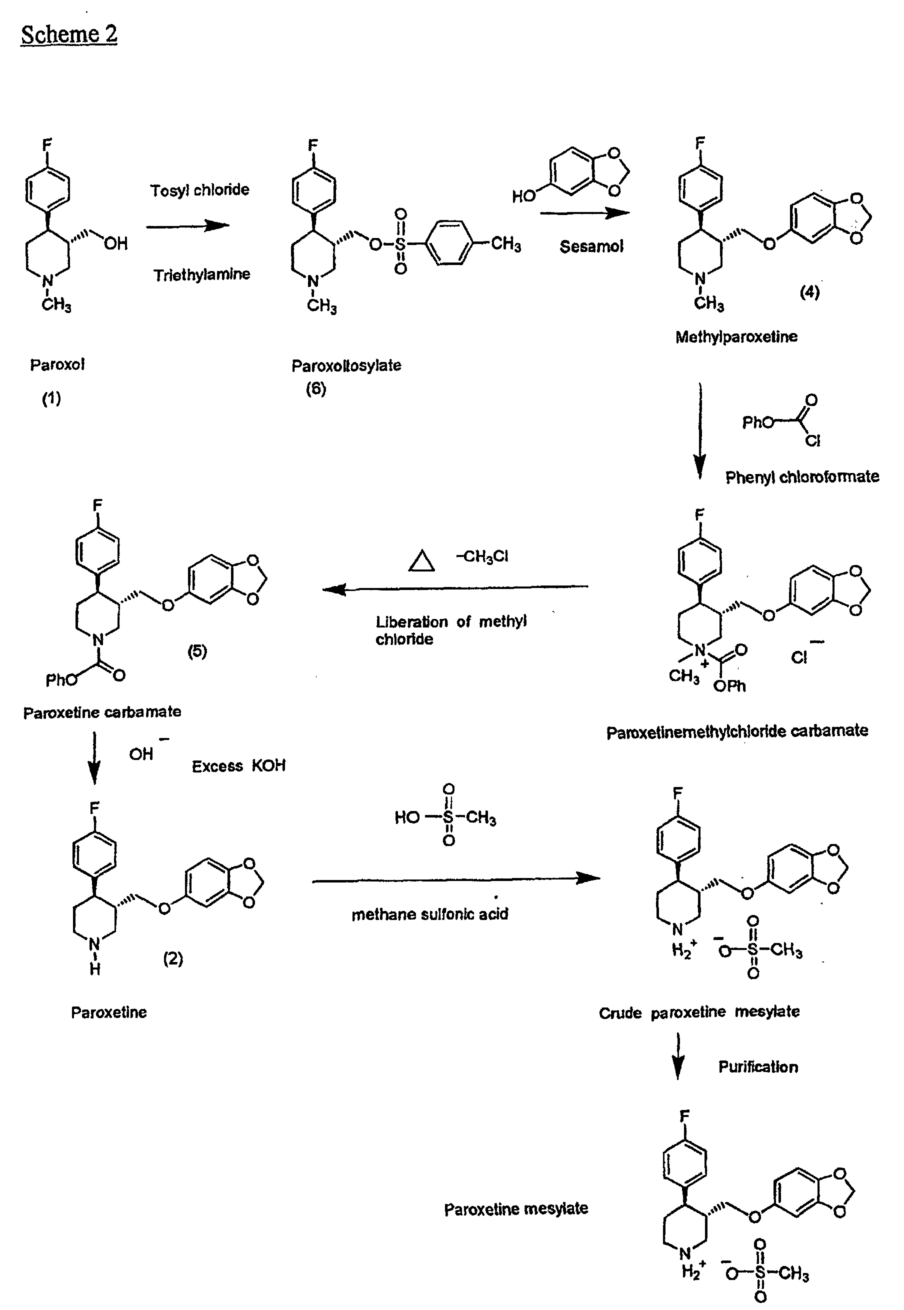

The compound (6) referred to as "paroxoltosylate" of the present invention is preparable by a process starting from a compound of formula (1), referred to as "paroxol" in this scheme.

The method of conversion of (1) to (6) preferably comprises the reaction of (1) in an inert solvent with p-toluenesulfonyl chloride (tosyl chloride). The resulting hydrogen chloride is trapped by a base, preferably an organic base such as triethylamine, pyridine and the like. The base is present in the reaction medium and usually it is used in a slight excess.

The solvent is preferably inert to tosylchloride in order to prevent solvolysis, and, should dissolve, at least partly, the reactants and should allow the product to crystallise therefrom. The choice of solvent cannot be derived from the prior art. The related prior art disclosures employ toluene, toluene/methylisobutylketone, chloroform, dichloromethane i.e. the solvents wherein the product is well soluble, and wherby water is used for the removal of the formed salt of the base present. As a result, the product does not crystallise and can only be obtained after evaporation of the solvent, mostly as an oily product. The oily nature of the products is most probably caused also by the residue of the solvent and water entrapped in the product.

The inventors have found that ethyl acetate is a suitable solvent, but that other solvents satisfying the above criteria are not to be excluded.

In the preferred process, the carbinol (1) "paroxol" in ethyl acetate can be reacted with tosyl chloride in the presence of 1.0-1.5 molar amount of triethylamine or pyridine at a temperature close to ambient or slightly elevated (from 0 to 80 C, preferably 20-70 C, most preferably at about 60 C). Care is to be taken that the solvent and reagents are substantially anhydrous. Triethylamine or pyridine hydrochloride resulting from the reaction is insoluble in the reaction medium and is easily removed after completation of the reaction by filtration, preferably at elevated temperature. After cooling the clear filtrate, the product (6) crystallises spontaneously. If desired, the clear filtrate may be slightly concentrated prior to crystallisation, e.g. on a rotary vacuum evaporator.

The precipitated compound (6) is isolated from the reaction mixture by filtration or centrifugation; washing the solid with appropriate washing liquid and drying the solid is recommended.

Dependent on the solvent system, isolation technique, washing and drying conditions, the compound (6) may be isolated as a hydrate or as a solvate or as an acid addition salt.

The compound (6) has been identified and characterised by one or more of the following procedures: elemental analysis, nuclear magnetic resonance spectroscopy (NMR), nfrared spectroscopy (IR) and high performance liquid chromatography (HPLC).

The starting compound (1), as discussed previously, usually contains a certain amount of the structurally related des-fluoro impurity which reacts the same way as (1) and, subsequently produces des-fluoro impurities structurally related to compounds (2)-(5) in the respective production steps of paroxetine synthesis.

The content of the des-fluoro impurity in (1) is undesirably high when (1) has been prepared by the most convenient hydride reduction method; raw (1) so prepared can contain 1-2% of said impurity.

When the reactive intermediate (3) produced from (1) is used in situ as in the prior art methods, it was found by the inventors that the content of desfluoro-impurity in the subsequent steps of paroxetine production remained basically the same as that in the starting (1). This is undesirable. On the contrary, the compound (6) according to the present invention, when isolated from the reaction medium as a solid, contains, without employing any purification technique, considerably less des-fluoro impurity than the starting compound (1). The content of the des-fluoro impurity may be further decreased by subsequent recrystallisation of (6) from a suitable solvent., The compound (6) is so far the most advantageous intermediate of the general formula (3) in this respect and it is significantly superior to any other similar intermediates as disclosed in the prior art.

Not all solvents were proved to be suitable for recrystallisation of (6) from the purification effect and yield points of view.

The inventors have shown that the choice of the solvent was not self evident and could not be deduced without experimentation. For instance acetone, though being a water miscible organic solvent, did not work.

Suitable solvents preferably comprise lower alkanols such as methanol, ethanol or most preferably isopropanol.

The inventors have shown that crystallisation of (6) from ethanol exhibited a purification effect (decrease of des-fluoro impurity) of 16-25% with 85-95% yield, isopropanol provided 35-40% effect in 85-90% yield. Ethyl acetate provided good yields in recrystallisation but with almost 0% purification effect.

If required, namely when the starting (1) contains considerably large amount of the des-fluoro impurity, the recrystallisation may be repeated.

The compound of formula (6) may be also isolated as, or converted to an acid addition salt. A good crystallising salt is the tosylate salt which may be prepared from (6) and p-toluene sulfonic acid in an aqueous medium and crystallised from the same medium in almost 100% yield. The purification effect of such conversion is still considerable, although lower (10-15%); it is however not excluded that a conversion to another acid addition salt will exhibit higher purification effect.

The above disclosed production method, optionally in combination with one or more recrystallisations, may provide the compound (6) and/or its acid addition salt with the content of less than 0.2% of the des-fluoro impurity. This particular quality of (6) allows the production of paroxetine in a pharmaceutically acceptable quality without the need of employing complicated, expensive and time consuming specific purification steps focused to des-fluoro impurity removal.

The amount of the structurally related des-fluoro impurities in products (1) and (6) can advantageously be monitored by HPLC, preferably using a reference substance of the des-fluoro impurity prepared according methods known *per se*.

The compound (6) is sufficiently stable towards heat, moisture and light so that it can be stored and transported at standard storage/transport conditions. It may be handled (weighed, packed, sampled, charged in the reaction vessel etc.) under common protective precautions, without need a special equipment.

The compound of formula (6), particularly in a solid state and optionally recrystallised, can be further converted into paroxetine of formula (2) for example employing procedures outlined in prior art disclosures, e.g. in US 4,007,196 and 4,721,723. The paroxetine may then be further processed to other pharmaceutically acceptable acid addition salts, such as paroxetine mesylate. An example of conversion of compound (1) to paroxetine mesylate via compound (6) of the present invention is shown in the scheme 2 above.

The following examples illustrate the invention but it should be understood that the present invention is by no means restricted to these specific examples.

### EXAMPLES

### Example 1

### (-)trans-4-(p-fluorophenyl)-3-(p-toluenesulfonyloxymethyl)-1-methyl-piperidine, compound (6)

5.2 g of (-)trans-4-(p-fluorophenyl) -N-methyl- piperidine 3-carbinol, "paroxol" was added under stirring to a mixture of 25 ml of ethyl acetate and 3.7 ml of triethylamine. After dissolution, the mixture was cooled to 0-5C and a solution of 4.7 g of p-toluene sulfochloride in 9.5 ml of ethyl acetate was added dropwise at the same temperature within 1 hour.

The reaction mixture was stirred at the same temperature for 1 hour and then for 24 hours at ambient temperature. The mixture was then heated to 60 C and filtered hot. The filtration cake was washed with 9 ml of ethyl acetate. The combined filtrates were concentrated in vacuo to approx. 25 ml volume, the concentrate was cooled under stirring to 0-5C and maintained at the same temperature for 24 hours.

The resulting crystals were filtered off, washed with 10 ml of cold ethyl acetate and dried. Yield 7.3 g.

The compound was identified by NMR and IR spectra, characterised by m.p. and optical rotation, the purity was determined by HPLC.

For the synthesis of the starting material, see e.g. US 4,902,801.

### Example 2

### Recrystallization of (-)trans-4-(p-fluorophenyl)-3-(p-toluenesulfonyloxymethyl)- 1-methyl piperidine, compound (6)

7.3 g of the compound from Example 1 was dissolved in 11.5 ml of isopropanol at 60C and the resulting solution was slowly cooled to 20C. When the crystallization started, the mixture was cooled to 0-5C and maintained at the same temperature for 24 hours. The solid product was filtered off, washed with 10 ml of cold isopropanol and dried. Yield: 6.6 g.

The invention having been described, it will be readily apparent to those skilled in the art that further changes and modifications in actual implementation of the concepts described herein can easily be made or may be learned by practice of the invention, without departing from the spirit and scope of the invention as defined by the following claims.

## Claims

1. Process for providing a compound of formula (6), 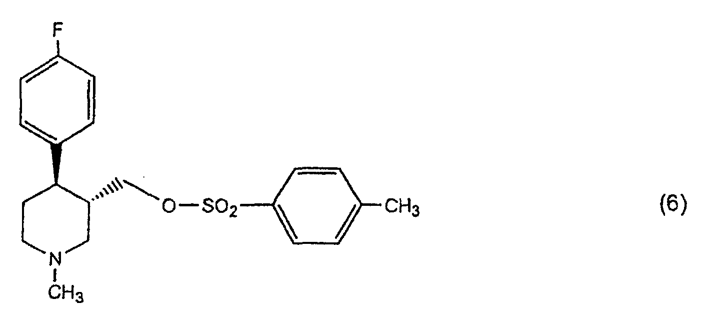 a hydrate, solvate, and/or salt thereof, for example a pharmaceutically acceptable acid addition salt, comprising the step of reacting a compound of formula (1): 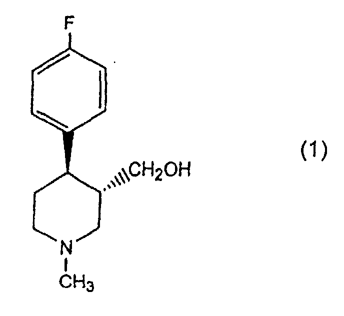 with a tosyl moiety, providing compound.

2. Process according to claim 1 wherein the tosyl moiety is provided by reacting the compound (1) with a tosyl-halide.

3. Process according to claim 2 wherein the tosyl halide is p-toluene sulfonyl chloride.

4. Process according to any of the claims 1-3 carried out in the presence of a base, preferably an organic base such as tri-ethylamine or pyridine, in a molar amount lying in the range of about 1-1.5.

5. Process according to any of the preceding claims carried out in the presence of a solvent, which solvent is preferably substantially inert to tosylchloride.

6. Process according to claim 5 wherein the solvent is ethyl acetate.

7. Process according to any of the preceding claims carried out at a temperature lying in the range 0-80, for example 20-70, and preferably about 60 degrees centigrade.

8. Process according to any of the preceding claims wherein compound (6) precipitates and is isolated in a solid state, preferably by filtration of the reaction mixture.

9. Process according to claim 8 wherein compound (6) is crystallised from an organic, water miscible solvent, preferably being an alkanol, preferably a lower alkanol and most preferably being isopropanol.

10. Process according to claim 8 and 9 wherein the isolated compound (6) contains less than about 0.2% of the des-fluoroimpurity.

11. Process according to any of the preceding claims wherein compound (6) is successively reacted with:
- sesamol,
- a haloformate, preferably being a chloroformate having a formula Cl-COOR, wherein R is an alkyl or an aryl group, preferably being a phenyl group,
- an alkali, preferably in excess and most preferably being KOH, to provide paroxetine, and optionally
- a pharmaceutically acceptable acid to convert paroxetine to a pharmaceutically acceptable acid addition salt, preferably paroxetine mesylate.

12. A process according to claims 8 and 9 wherein the produced paroxetine and/or its pharmaceutically acceptable acid addition salt contains less than 0.1% of the des-fluoroimpurity.

13. A compound (-)trans-4-(p-fluorophenyl)-3-(p-toluenesulfonyloxymethyl)-1-methyl-piperidine of the formula (6), 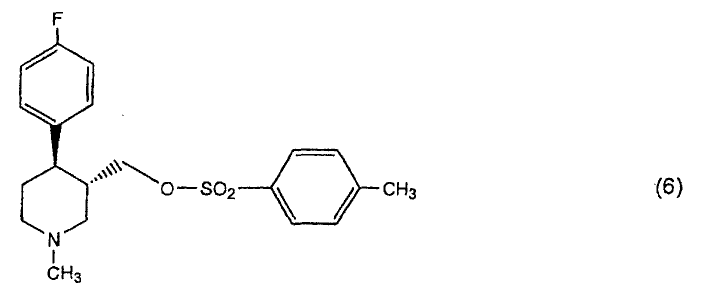 or salt thereof, preferably a pharmaceutically acceptable acid addition salt.

14. Compound according to claim 13 being in a solid state, a hydrate thereof or a solvate thereof.

15. Compound according to claim 14 being in a crystalline state.

16. Compound according to any of the preceding claims 13-15, comprising less than about 0.2% of a des-fluoro impurity thereof.

17. Paroxetinemethylchloride carbamate having the following structure:

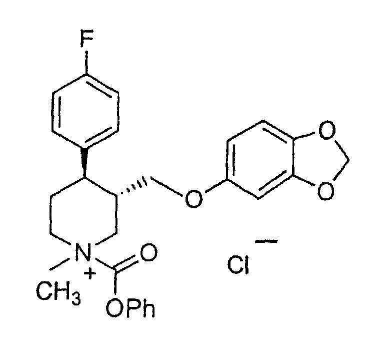

18. Paroxetine carbamate having the following structure:

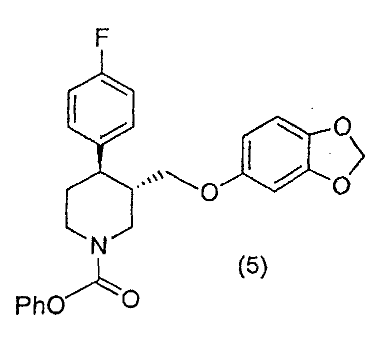

19. Compound according to claims 13-18 obtainable according to any of the claims 1-12.

20. Compound according to any of the claims 13-19, being in a solid, preferably crystalline state, preferably comprising about 0.2% or less of a des-fluoro impurity.

21. Use of any of the compounds according to any of the claims 13-20 in the production of paroxetine, and/or paroxetine mesylate.

22. Use of ethyl acetate as a solvent for a compound of formula (1) as referred to in claim 1.

## Patentansprüche

1. Verfahren zur Bereitstellung einer Verbindung der Formel (6), 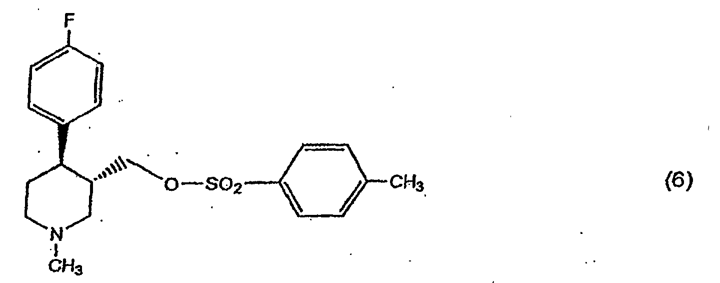 eines Hydrats, eines Solvats und/oder eines Salzes davon, beispielsweise eines pharmazeutisch annehmbaren Säureadditionssalzes, das die Stufe umfasst, bei der eine Verbindung der Formel (1) 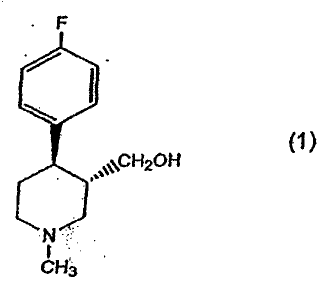 mit einer Tosyleinheit unter Herstellung der Verbindung umgesetzt wird.

2. Verfahren nach Anspruch 1, worin die Tosyleinheit durch Umsetzen der Verbindung (1) mit einem Tosylhalogenid bereitgestellt wird.

3. Verfahren nach Anspruch 2, worin das Tosylhalogenid p-Toluolsulfonylchlorid ist.

4. Verfahren nach einem der Ansprüche 1 bis 3, das in Gegenwart einer Base, bevorzugt einer organischen Base, wie Triethylamin oder Pyridin, in einer Molmenge, die im Bereich von etwa 1-1,5 liegt, durchgeführt wird.

5. Verfahren nach einem der vorangegangenen Ansprüche, das in Gegenwart eines Lösungsmittels, welches Lösungsmittel bevorzugt im wesentlichen inert gegenüber Tosylchlorid ist, durchgeführt wird.

6. Verfahren nach Anspruch 5, worin das Lösungsmittel Ethylacetat ist.

7. Verfahren nach einem der vorangegangenen Ansprüche, das bei einer Temperatur, die im Bereich von 0-80, beispielsweise 20-70 und bevorzugt etwa 60°C liegt, durchgeführt wird.

8. Verfahren nach einem der vorangegangenen Ansprüche, worin die Verbindung (6) ausgefällt wird und im festen Zustand isoliert wird, bevorzugt durch Filtration des Reaktionsgemischs.

9. Verfahren nach Anspruch 8, worin die Verbindung (6) aus einem organischen, wassermischbaren Lösungsmittel, das bevorzugt ein Alkanol ist, bevorzugt ein niedrigmolekulares Alkanol und am meisten bevorzugt Isopropanol ist, kristallisiert wird.

10. Verfahren nach Anspruch 8 und 9, worin die isolierte Verbindung (6) weniger als etwa 0,2 % der Defluorverunreinigung enthält.

11. Verfahren nach einem der vorangegangenen Ansprüche, worin die Verbindung (6) nacheinander umgesetzt wird mit:
- Sesamol,
- einem Halogenformat, das bevorzugt ein Chlorformat der Formel Cl-COOR ist, worin R eine Alkyl- oder eine Arylgruppe, bevorzugt eine Phenylgruppe, bedeutet,
- einer Alkalie, bevorzugt im Überschuss, die am meisten bevorzugt KOH ist, um Paroxetin herzustellen und wahlweise,
- einem pharmazeutisch annehmbaren Salz, um das Paroxetin in ein pharmazeutisch annehmbares Säureadditionssalz, bevorzugt Paroxetinmesylat, umzuwandeln.

12. Verfahren nach Anspruch 8 und 9, worin das hergestellte Paroxetin und/oder sein pharmazeutisch annehmbares Säureadditionssalz weniger als 0,1 % der Defluorverunreinigung enthält.

13. Verbindung (-)trans-4-(p-Fluorphenyl)-3-(p-toluolsulfonyloxymethyl)-1-methylpiperidin der Formel (6) 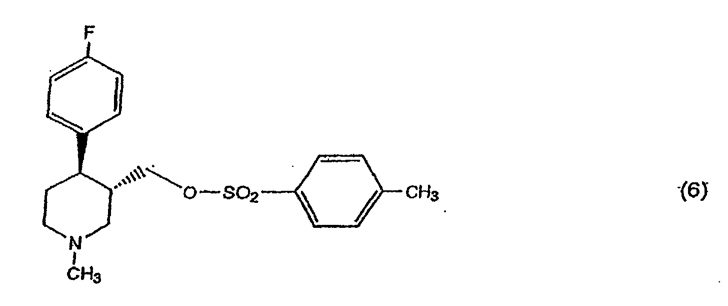 oder ein Salz davon, vorzugsweise ein pharmazeutisch annehmbares Säureadditionssalz.

14. Verbindung nach Anspruch 13, das im festen Zustand ein Hydrat davon oder ein Solvat davon darstellt.

15. Verbindung nach Anspruch 14, das im kristallinen Zustand vorliegt.

16. Verbindung nach einem der vorangegangenen Ansprüche 13 - 15, die weniger als etwa 0,2 % der Defluorverunreinigung davon umfasst.

17. Paroxetinmethylchloridcarbamat mit der folgenden Struktur:

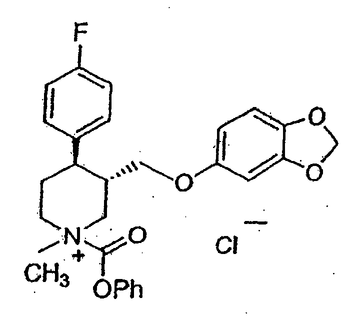

18. Paroxetincarbamat mit der folgenden Struktur:

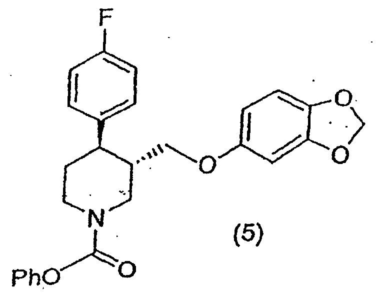

19. Verbindung nach den Ansprüchen 13 - 18, die nach einem der Ansprüche 1 - 12 erhältlich ist.

20. Verbindung nach einem der Ansprüche 13 bis 19, die in einem festen, vorzugsweise kristallinen Zustand vorliegt und bevorzugt etwa 0,2 % oder weniger einer Defluorverunreinigung umfasst.

21. Verwendung einer der Verbindungen nach einem der Ansprüche 13 - 20 bei der Herstellung von Paroxetin und/oder Paroxetinmesylat.

22. Verwendung von Ethylacetat als Lösungsmittel für eine Verbindung der Formel (1), auf die in Anspruch 1 Bezug genommen wird.

## Revendications

1. Procédé de production d'un composé de formule (6), 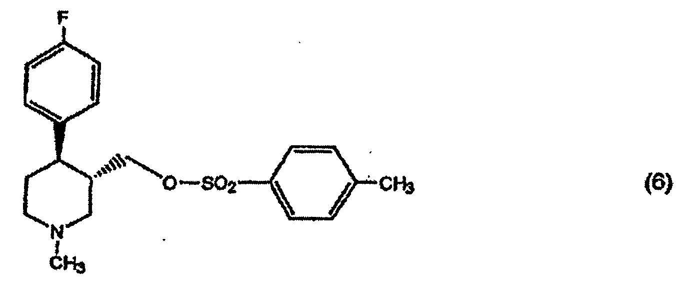 d'un hydrate, d'un solvat, et/ou d'un sel de celui-ci, par exemple un sel d'addition acide pharmaceutiquement acceptable, comprenant l'étape consistant à faire réagir un composé de formule (1) : 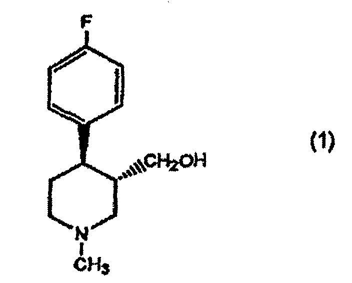 avec un composé fournissant un radical tosyle.

2. Procédé selon la revendication 1, dans lequel le radical tosyle est fourni en faisant réagir le composé (1) avec un halogénure de tosyle.

3. Procédé selon la revendication 2, dans lequel l'halogénure de tosyle est le chlorure de p-toluène sulfonyle.

4. Procédé selon l'une quelconque des revendications 1 à 3, réalisé en présence d'une base, de préférence une base organique telle que la triéthylamine ou la pyridine, en quantité molaire se situant dans la plage d'environ 1 à 1,5.

5. Procédé selon l'une quelconque des revendications précédentes, réalisé en présence d'un solvant, lequel solvant est de préférence essentiellement inerte vis-à-vis du chlorure de tosyle.

6. Procédé selon la revendication 5, dans lequel le solvant est l'acétate d'éthyle.

7. Procédé selon l'une quelconque des revendications précédentes, réalisé à une température se situant dans la plage de 0 à 80, par exemple 20 à 70, et de préférence environ 60 degrés centigrades.

8. Procédé selon l'une quelconque des revendications précédentes, dans lequel le composé (6) précipite et est isolé sous un état solide, de préférence par filtration du mélange réactionnel.

9. Procédé selon la revendication 8, dans lequel le composé (6) est cristallisé à partir d'un solvant organique miscible à l'eau, étant, de préférence, un alcanol, de préférence un alcanol inférieur et étant, de manière préférée entre toutes, l'isopropanol.

10. Procédé selon les revendications 8 et 9, dans lequel le composé (6) isolé contient moins d'environ 0,2% d'impuretés desfluoro.

11. Procédé selon l'une quelconque des revendications précédentes, dans lequel le composé (6) est successivement mis à réagir avec :
du sésamol,
un halogéno-formate, étant de préférence un chloroformate ayant une formule Cl-COOR, dans laquelle R est un groupe alkyle ou aryle, étant, de préférence, un groupe phényle,
un alcali, de préférence en excès et étant, de manière préférée entre toutes, du KOH pour produire la paroxétine, et éventuellement
un acide pharmaceutiquement acceptable pour convertir la paroxétine en un sel d'addition acide pharmaceutiquement acceptable, de préférence le mésylate de paroxétine.

12. Procédé selon les revendications 8 et 9, dans lequel la paroxétine produite et/ou son sel d'addition acide pharmaceutiquement acceptable contient moins de 0,1% d'impuretés desfluoro.

13. Composé de (-)trans-4-(p-fluorophényl)-3-(p-toluène-sulfonyloxyméthyl)-1-méthyl-pipéridine de formule (6), 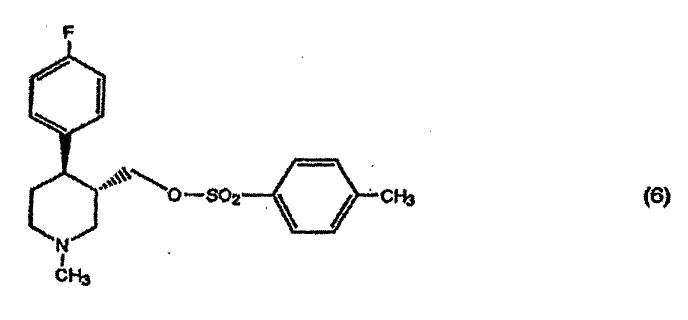 ou un sel de celui-ci, de préférence un sel d'addition acide pharmaceutiquement acceptable.

14. Composé selon la revendication 13, étant sous un état solide, un hydrate de celui-ci ou un solvat de celui-ci.

15. Composé selon la revendication 14, étant sous un état cristallin.

16. Composé selon l'une quelconque des revendications précédentes 13 à 15, comprenant moins d'environ 0,2% d'impuretés desfluoro de celui-ci.

17. Carbamate de paroxétine méthylchlorure ayant la structure suivante :

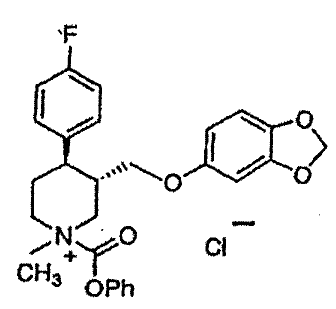

18. Carbamate de paroxétine ayant la structure suivante :

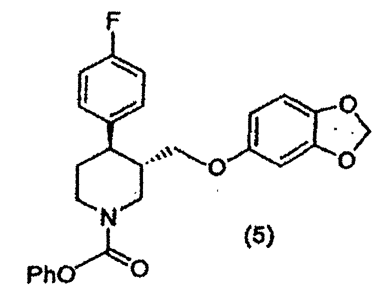

19. Composé selon les revendications 13 à 18, pouvant être obtenu selon l'une quelconque des revendications 1 à 12.

20. Composé selon l'une quelconque des revendications 13 à 19, étant, sous un état solide, de préférence, cristallin, comprenant, de préférence, environ 0,2% ou moins d'impuretés desfluoro.

21. Utilisation de l'un quelconque des composés selon l'une quelconque des revendications 13 à 20, dans la production de paroxétine et/ou de mésylate de paroxétine.

22. Utilisation de l'acétate d'éthyle comme solvant pour un composé de formule (1) tel que présenté dans la revendication 1.
